# EUROPEAN PATENT APPLICATION

(11) **EP 2 808 033 A1**
(43) Date of publication of application: **03.12.2014**
(21) Application number: 14174866.5
(22) Date of filing: 18.11.2005
(51) Int. Cl.: A61K 39/395, C07K 16/28, A61P 25/28

(54) **Treatment for multiple sclerosis**

(30) Priority: 19.11.2004 US 629700 P
(62) Divisional of application: 05824954.1
(71) Applicant: Biogen Idec MA Inc., Cambridge, Massachusetts 02142 (US)
(72) Inventor: Panzara, Michael, Winchester, MA Massachusetts 01890 (US); Sandrock, Alfred, Newton, MA Massachusetts 02459 (US)
(74) Representative: Miller, David James

(57) **Abstract**

Methods of treating multiple sclerosis and other disorders are disclosed. Subjects suffering from multiple sclerosis are treated with an anti-VLA-4 antibody, e.g., natalizumab.

## Description

### RELATED APPLICATIONS

This application claims the benefit of U.S. Provisional Application No. 60/629,700 filed November 19,2004, the entire contents of which are hereby incorporated by reference herein.

### BACKGROUND

Multiple sclerosis (MS) is one of the most common diseases of the central nervous system. Today over 2,500,000 people around the world have MS. Interferon beta and copaxone are the leading disease modifying therapies to treat MS currently on the market. Interferon beta therapy can decrease the relapse rate by 32-33% in MS patients (Jacobs et al., Ann Neurol. 39:285-94, 1996; see also Neurology 43:641-643, 1993).

### SUMMARY OF THE INVENTION

The invention is based, at least in part, on the findings that a VLA-4 blocking therapy, e.g., a VLA-4 binding antibody, e.g., natalizumab, is safe and effective for use in treating a patient who has previously been treated for an inflammatory disorder, e.g., multiple sclerosis. In particular, administration of natalizumab led to a dramatic improvement in the condition of patients with MS, who previously demonstrated an inadequate response to a prior treatment, e.g., a prior biologic therapy, in particular, prior interferon beta therapy. Accordingly, in one aspect, this disclosure features a method of treating a subject, e.g., a human subject, having an inflammatory disorder, and who exhibits an inadequate response to a first therapy, e.g., a first agent, e.g., an agent described herein, e.g., interferon beta. The inflammatory disorder can be, e.g., MS, rheumatoid arthritis, inflammatory bowel disease, or systemic lupus erythematosus. The method includes administering to the subject a VLA-4 binding protein, e.g., a VLA-4 binding antibody, e.g., natalizumab, in an amount and for a time sufficient to treat the disorder. The VLA-4 binding protein, e.g., VLA-4 binding antibody, e.g., natalizumab, can be administered at least once, e.g., at least 2, 3, 4, 5 or more times, when the first agent, e.g., interferon beta, is not present at a therapeutic level in the subject. (Unless otherwise stated, a VLA-4 binding antibody, preferably natalizumab, is the preferred VLA-4 binding protein in all embodiments described herein. Similarly, unless stated otherwise, the preferred disorder is multiple sclerosis.)

In one embodiment, the administration of the VLA-4 binding protein, e.g., VLA-4 binding antibody, e.g., natalizumab, is continued, in the absence of administration of the first agent, e.g., interferon beta, for at least 2 months, e.g., for at least 4, 8, 12, 24, or 48 months.

In one embodiment, the first therapy, e.g., the first agent, e.g., interferon beta, is not administered to the subject after a first administration of the VLA-4 binding protein, e.g., VLA-4 binding antibody, e.g., natalizumab. In another embodiment, the administration of the VLA-4 binding protein, e.g., VLA-4 binding antibody, e.g., natalizumab, replaces the administration of the first agent, e.g., interferon beta.

An inadequate response can be, e.g., a response that is less than a predetermined level of response, as described herein. An inadequate response can also be an adverse reaction or an intolerable or unacceptable toxic response to a first therapy, e.g., a first agent, e.g., interferon beta.

In one embodiment, the subject is one to whom a first therapy, e.g., a first agent, e.g., interferon beta, was previously administered for at least 3 months, e.g., at least 6, 12, 18, or 24 months.

In one embodiment, the subject has not previously been administered a VLA-4 binding protein, e.g., VLA-4 binding antibody, e.g., natalizumab, since being diagnosed with the disorder, e.g., MS.

In some embodiments, the subject has not been administered the first agent, e.g., interferon beta, for at least 1 month, e.g., at least 2, 3, 4, 5, 6, 12, or 24 months, prior to a first administration of the VLA-4 binding protein, e.g., VLA-4 binding antibody, e.g., natalizumab.

In other embodiments, the subject has been administered the first agent, e.g., interferon beta, within 6 weeks, e.g., within 4, 2 or 1 week, or within 6, 5, 4, 3, 2 or 1 day, prior to a first administration of the VLA-4 binding protein, e.g., VLA-4 binding antibody, e.g., natalizumab.

In one embodiment, the subject has not been administered mitoxantrone, cyclophosphamide, cyclosporine, azathioprine or methotrexate within 3 months, e.g., within 6, 12, 18, 24, 30 or 36 months, of a first administration of the VLA-4 binding protein, e.g., VLA-4 binding antibody, e.g., natalizumab.

In one embodiment, the subject is an adult, e.g., a subject whose age is greater or equal to 16, 18, 19, 20, 24, or 30 years. Typically, the subject is between 19 and 55 years of age. The subject can be female or male.

In one embodiment, the subject has relapsing remitting multiple sclerosis. In another embodiment, the subject has chronic progressive multiple sclerosis, e.g., primary-progressive (PP), secondary progressive, or progressive relapsing multiple sclerosis.

In one embodiment, the subject has an inflammatory disorder, e.g., MS, that is refractory to the first therapy, e.g., the first agent, e.g., interferon beta.

In one embodiment, the VLA-4 binding protein is a VLA-4 binding antibody, e.g., a full length antibody such as an IgG1, IgG2, IgG3, or IgG4. Typically the antibody is effectively human, human, or humanized. The VLA-4 binding antibody can inhibit VLA-4 interaction with a cognate ligand of VLA-4, e.g., VCAM-1. The VLA-4 binding antibody binds to at least the α chain of VLA-4, e.g., to the extracellular domain of the α4 subunit. For example, the VLA-4 binding antibody recognizes epitope B (e.g., B1 or B2) on the α chain of VLA-4. The VLA-4 binding antibody may compete with, or have an overlapping epitope with, natalizumab, HP1/2, or another VLA-4 binding antibody described herein for binding to VLA-4. In a preferred embodiment, the VLA-4 binding antibody is natalizumab or includes the heavy chain and light chain variable domains of natalizumab.

The first therapy, e.g., first agent, can be a biologic, e.g., a protein of defined sequence such as an interferon. In one embodiment, the first agent includes an interferon beta, e.g., interferon beta-1a, e.g., AVONEX® (interferon beta-1a) or Rebif® (interferon beta-1a), or BETASERON® (interferon beta-1b). The first agent can also be a protein of undefined sequence, e.g., a random copolymer of selected amino acids, e.g., glatiramer acetate.

In one embodiment, the VLA-4 binding protein, e.g., VLA-4 binding antibody, e.g., natalizumab, is administered at a dose sufficient to achieve at least 80% (preferably 90%, 95%, 100%, 110% or greater) of the bioavailability achieved with a monthly (e.g., once every four weeks) dose of between about 50 and 600 mg (e.g., between about 200 and 400 mg, e.g., about 300 mg by intravenous (IV) route). For example, the VLA-4 binding antibody is administered as a monthly IV infusion of between about 50 and 600 mg (e.g., between about 200 and 400 mg, e.g., about 300 mg). In another example, the VLA-4 binding antibody is administered as a subcutaneous (SC) injection of between 25-300 mg (e.g., between 50 and 150 mg, e.g., about 75 mg), e.g., once a week, twice a week or once every 2 weeks.

In some embodiments, the VLA-4 binding protein, e.g., VLA-4 binding antibody, e.g., natalizumab, can be administered in an amount that is effective to result in one or more of the following: a) decreased severity of relapse, b) prevention of an increase in EDSS score, c) decreased EDSS score (e.g., a decrease of greater than 1, 1.5, 2, 2.5, or 3 points, e.g., over at least six months, one year, or longer), d) decreased number of new Gd+ lesions, e) reduced rate of appearance of new Gd+ lesions, and f) decreased increase in Gd+ lesion area. The VLA-4 binding protein, e.g., natalizumab, can be administered in multiple doses and in an amount that is effective to maintain an exacerbation-free or relapse free period for at least three, six, or nine months, or one, two, or three years.

In some embodiments, a subject is evaluated, e.g., for indicia of responsiveness, after receiving the VLA-4 binding protein, e.g., natalizumab. A skilled artisan can use one or more various clinical or other indicia of effectiveness of treatment, e.g., EDSS score; MRI scan; relapse number, rate, or severity; multiple sclerosis functional composite (MSFC); multiple sclerosis quality of life inventory (MSQLI). The subject can be monitored at various times during a regimen. In one embodiment, the subject is not examined for interferon bioavailability (e.g., before or after the administering).

In another aspect, the disclosure features a method of treating a subject, e.g., a human subject, having an inflammatory disorder, e.g., multiple sclerosis. The method includes selecting a subject on the basis of having an inadequate response to treatment with a first agent for multiple sclerosis (e.g., the MS is refractory to the first agent); and administering to the subject a VLA-4 binding protein, e.g., a VLA-4 binding antibody, e.g., natalizumab, in an amount and for a time sufficient to treat the disorder. The VLA-4 binding protein, e.g., VLA-4 binding antibody, e.g., natalizumab, can be administered at least once, e.g., at least 2, 3, 4, 5 or more times, when the first agent, e.g., interferon beta, is not present at a therapeutic level in the subject.

In one embodiment, the method also includes determining whether the subject has a disorder, e.g., multiple sclerosis, that is refractory to treatment with a first agent, e.g., interferon beta. In one embodiment, determining whether the disorder is refractory includes evaluating the level of response, e.g., evaluating a response described herein, to treatment with the first agent, e.g., interferon beta, and if the subject has failed to meet a predetermined level of response to the treatment, then determining that the subject has a disorder, e.g., MS, that is refractory to treatment with the first agent, e.g., interferon beta.

The method can also include any of the other embodiments described herein.

In another aspect, the disclosure features a method of treating a subject, e.g., a human subject, having an inflammatory disorder, e.g., multiple sclerosis. The method includes determining if the subject has failed to meet a predetermined level of response, e.g., a response described herein, to treatment with a first agent, e.g., an agent described herein (but not a VLA-4 binding protein, e.g., natalizumab), or has exhibited an adverse reaction or intolerable or unacceptable toxicity. The method includes administering to the subject a VLA-4 binding protein, e.g., natalizumab, in an amount and for a time sufficient to treat the disorder, if the subject has failed to meet the predetermined level of response to the treatment, or has exhibited an adverse reaction or intolerable or unacceptable toxicity. The VLA-4 binding protein, e.g., natalizumab, can be administered at least once, e.g., at least 2, 3, 4, 5 or more times, when the first agent, e.g., interferon beta, is not present at a therapeutic level in the subject.

The method can also include any of the other embodiments described herein.

In another aspect, the disclosure features a method of treating a subject, e.g., a human subject, having an inflammatory disorder, e.g., multiple sclerosis. The method includes selecting a subject who has failed to meet a predetermined level of response, e.g., a response described herein, to treatment with a first agent, e.g., an agent described herein (but not a VLA-4 binding protein, e.g., natalizumab), or has exhibited an adverse reaction or intolerable or unacceptable toxicity. The method includes administering to the subject a VLA-4 binding protein, e.g., natalizumab, in an amount and for a time sufficient to treat the disorder, if the subject has failed to meet the predetermined level of response to the treatment, or has exhibited an adverse reaction or intolerable or unacceptable toxicity. The VLA-4 binding protein, e.g., natalizumab, can be administered at least once, e.g., at least 2, 3, 4, 5 or more times, when the first agent, e.g., interferon beta, is not present at a therapeutic level in the subject.

The method can also include any of the other embodiments described herein.

In another aspect, the disclosure features a method of treating a subject, e.g., a human subject, having an inflammatory disorder, e.g., multiple sclerosis. The method includes administering to the subject a VLA-4 binding protein, e.g., natalizumab, in an amount and for a time sufficient to treat the disorder. The subject can be a subject who has failed to meet a predetermined level of response, e.g., a response described herein, to treatment with a first agent, e.g., an agent described herein (but not a VLA-4 binding protein, e.g., natalizumab), or has exhibited an adverse reaction or intolerable or unacceptable toxicity. The VLA-4 binding protein, e.g., natalizumab, can be administered at least once, e.g., at least 2,3,4, 5 or more times, when the first agent, e.g., interferon beta, is not present at a therapeutic level in the subject.

The method can also include any of the other embodiments described herein.

In another aspect, the disclosure features a method of treating a subject, e.g., a human subject, having an inflammatory disorder, e.g., multiple sclerosis. The method includes administering to the subject a first agent, e.g., interferon beta, and evaluating the subject for response, e.g., a response described herein, to treatment with the first agent, e.g., interferon beta. The method also includes administering to the subject a VLA-4 binding protein, e.g., natalizumab, in an amount and for a time sufficient to treat the disorder, if the subject has failed to meet the predetermined level of response to the treatment, or has exhibited an adverse reaction or intolerable or unacceptable toxicity. The VLA-4 binding protein, e.g., natalizumab, can be administered at least once, e.g., at least 2, 3, 4, 5 or more times, when the first agent, e.g., interferon beta, is not present at a therapeutic level in the subject.

The method can also include any of the other embodiments described herein.

In another aspect, the disclosure features a method of evaluating a subject, e.g., a human subject, having an inflammatory disorder, e.g., multiple sclerosis, for effectiveness of treatment of the disorder, e.g., multiple sclerosis. The method includes selecting a subject who has previously failed to meet a predetermined level of response, e.g., a response described herein, to treatment with a first agent, e.g., an agent described herein (but not a VLA-4 binding protein, e.g., natalizumab), or has previously exhibited an adverse reaction or intolerable or unacceptable toxicity. The subject is one who has subsequently received a VLA-4 binding protein, e.g., natalizumab, at least once, e.g., at least 2, 3, 4, 5 or more times, when the first agent, e.g., interferon beta, was not present at a therapeutic level in the subject. The method also includes determining the level of response, e.g., a response described herein, e.g., a parameter associated with the disorder or a side effect, to the treatment with the VLA-4 binding protein, e.g., natalizumab.

The method can also include any of the other embodiments described herein.

In another aspect, the disclosure features a method of treating a subject having multiple sclerosis who, while being treated with interferon beta for a specified period of time, e.g., for at least 3 months, 6 months, 9 months, or at least one year, has had one or more of: (a) at least one relapse, (b) appearance of new MRI enhancing lesions, and (c) progression of disability (EDSS). The method includes administering to the subject a VLA-4 binding protein, e.g., a VLA-4 binding antibody, e.g., natalizumab, in an amount and for a time sufficient to treat the disorder. The VLA-4 binding protein, e.g., VLA-4 binding antibody, e.g., natalizumab, can be administered at least once, e.g., at least 2,3, 4, 5 or more times, when the first agent, e.g., interferon beta, is not present at a therapeutic level in the subject.

The method can also include any of the other embodiments described herein.

In another aspect, the invention features, a method of making a decision, e.g., a medical or financial decision. The method includes:
generating or receiving data on the response, e.g., a response described herein, to treatment with a first agent, e.g., interferon beta, of a subject having an inflammatory disorder, e.g., multiple sclerosis, (e.g., receiving the response data generated by a method described herein); and
using the data to make the decision, e.g., selecting between a first course of action and a second course of action.

In a preferred embodiment, the decision includes comparing the data to a standard and making the decision based on the relationship of the data to the standard. For example, the data can be a value or other term for the likelihood of response and if the value or other term has a preselected relationship to the standard, e.g., if the value or term in the data is greater than a reference standard, selecting a first course of action and if the data is less than a reference standard selecting a second course of action. A course of action can be, e.g., providing or not providing service or treatment, e.g., a VLA-4 binding protein, e.g., natalizumab, or paying for or not paying for all or part of a service or treatment, e.g., natalizumab.

In a preferred embodiment, the first course of action is suggesting or providing a first course of medical treatment, e.g., treatment with a VLA-4 binding protein, e.g., natalizumab, and the second course of action is suggesting or deciding that the treatment not be given or not providing the treatment.

In a preferred embodiment the first course of action includes or results in the authorization or transfer of funds to pay for a service or treatment, e.g., treatment with a VLA-4 binding protein, e.g., natalizumab, provided to a subject and the second course of action includes or results in the refusal to pay for a service or treatment provided to a subject. For example, an entity, e.g., a hospital, caregiver, government entity, or an insurance company or other entity, that pays for, or reimburses, medical expenses can use the outcome of a method described herein to determine whether a party, e.g., a party other than the subject patient, will pay for services or treatment provided to the patient. For example, a first entity, e.g., an insurance company, can use the outcome of a method described herein to determine whether to provide financial payment to, or on behalf of, a patient, e.g., whether to reimburse a third party, e.g., a vendor of goods or services, a hospital, a physician, or other care-giver, for a service or treatment provided to a patient.

### Definitions

As used herein, an "inadequate response" refers to a response that, as assessed by a patient or a skilled clinician, exhibits insufficient efficacy or intolerable or unacceptable toxicity. Insufficient efficacy can be defined by failure to meet a predetermined level of response to treatment with a first agent. In the case of MS, for example, insufficient efficacy may be defined as failure to exhibit at least a 10%, 20%, 30%, 40%, 50% or more decrease in one or more of: rate of relapse over time (e.g., relative to a standard, e.g., rate of relapse over time prior to treatment); severity of relapse; EDDS score; and MRI-enhancing lesion load, volume or new lesions; or other parameter disclosed herein. Intolerable toxicity can be an adverse reaction to a first agent that results in medical need or recommendation to discontinue use of the first agent. Examples of intolerable or unacceptable toxicity may include hepatic injury or dysfunction, severe allergic reaction, severe depression or suicidal ideation, anaphylaxis, or injection site necrosis. The methods described herein can reduce the occurrence and/or severity of such toxic effects.

In one embodiment, the subject has MS that is refractory to a first therapy, e.g., the subject has not shown a clinically acceptable or significant improvement in response to the first therapy, or initially showed an improvement in response to the first therapy but no longer demonstrates an improvement, as assessed by a standard clinical measure for the specific disorder.

For example, with regard to MS, a predetermined level of response to treatment with a first agent can be one or more of the following: a) at least 10%, 20%, 30%, 40%, 50%, 60% decreased rate of relapse over time (e.g., over 3 months, 6 months, 9 months, one year, 2 years, 3 years, 4 years or longer), e.g., compared to a reference value; b) at least 10%, 20%, 30%, 40%, 30%, 60% decreased severity of relapse; c) prevention of an increase in EDSS score; d) decreased EDSS score (e.g., a decrease of greater than 0.5, 1, 1.5,2, 2.5, or 3 points, e.g., over at least six months, one year, or longer); e) decreased number of new lesions (e.g., Gd+ lesions), e.g., at least 10%, 20%, 30%, 40%, 50%, 60% decreased with regard to a reference number of new lesions. The reference number of new lesions can be, e.g., the number of new lesions that appear over a similar time period in the subject under the previous therapy, or an average number expected for a subject having a similar stage or severity of the disorder in the absence of treatment or under the previous treatment; f) reduced rate of appearance of new lesions (e.g., Gd+ lesions), e.g., at least 10%, 20%, 30%, 40%, 50%, 60% reduced rate as compared to a reference rate of appearance of new lesions. The reference rate of appearance of new lesions can be, e.g., the rate of appearance in the subject under the previous therapy, or an average rate expected for a subject having a similar stage or severity of the disorder in the absence of treatment or under the previous treatment; and g) decreased increase in lesion area (e.g., Gd+ lesion area), e.g., as compared to a reference value of increase in lesion area. The reference value of increase in lesion area can be, e.g., the increase in Gd+ lesion area in the subject under the previous therapy, or an average increase expected for a subject having a similar stage or severity of the disorder in the absence of treatment or under the previous treatment.

In one embodiment, the subject is a breakthrough patient, i.e., a patient that has had one or more of: (a) at least one relapse; (b) appearance of new MRI enhancing lesions; and (c) progression of disability (EDSS) while being treated with the first therapy for a specified period of time, e.g., for at least 3 months, 6 months, 9 months, or at least one year.

In one embodiment, the subject has had less than a 40%, 30%, 25%, 20%, 15%, 10%, 5% decrease in relapse rate over time (e.g., over 6 months, 9 months, a year, 2 years, 3 years, 4 years or more) while on the first therapy. In some embodiments, the subject, after beginning VLA-4 binding agent therapy (e.g., administration of a VLA-4 binding antibody, e.g., natalizumab), can achieve at least 40%, 45%, 50%, 60%, 65%, 70% or greater decrease in relapse rate over time (e.g., over 6 months, 9 months, a year, 2 years, 3 years, 4 years or more).

In one embodiment, the subject has had less than a 50%, 40%, 30%, 25%, 20%, 15%, 10%, 5% decrease in new lesions (e.g., Gd+ or T2 hypertense lesions) or lesion volume over time (e.g., over 6 months, 9 months, a year, 2 years, 3 years, 4 years or more) while on the first therapy. In some embodiments, the subject, after beginning VLA-4 binding agent therapy (e.g., administration of a VLA-4 binding antibody, e.g., natalizumab), can achieve at least 50%, 60%, 65%, 70%, 80% or greater decrease in new lesions (e.g., Gd+ or T2 hypertense lesions) or lesion volume over time (e.g., over 6 months, 9 months, a year, 2 years, 3 years, 4 years or more).

The term "treating", as used herein, refers to administering a therapy in an amount, manner (e.g., schedule of administration), and/or mode (e.g., route of administration), effective to improve a disorder or a symptom thereof, or to prevent or slow the progression of a disorder or a symptom thereof. This can be evidenced by, e.g., an improvement in a parameter associated with a disorder or a symptom thereof, e.g., to a statistically significant degree or to a degree detectable to one skilled in the art. An effective amount, manner, or mode can vary depending on the subject and may be tailored to the subject. By preventing or slowing progression of a disorder or a symptom thereof, a treatment can prevent or slow deterioration resulting from a disorder or a symptom thereof in an affected or diagnosed subject.

The term "biologic" refers to a protein-based therapeutic agent. In a preferred embodiment, the biologic is at least 10, 20, 30, 40, 50 or 100 amino acid residues in length.

A "VLA-4 binding agent" refers to any compound that binds to VLA-4 integrin with a K_{d} of less than 10⁻⁶ M. An example of a VLA-4 binding agent is a VLA-4 binding protein, e.g., a VLA-4 binding antibody such as natalizumab.

A "VLA-4 antagonist" refers to any compound that at least partially inhibits an activity of a VLA-4 integrin, particularly a binding activity of a VLA-4 integrin or a signaling activity, e.g., ability to transduce a VLA-4 mediated signal. For example, a VLA-4 antagonist may inhibit binding of VLA-4 to a cognate ligand of VLA-4, e.g., a cell surface protein such as VCAM-1, or to an extracellular matrix component, such as fibronectin or osteopontin. A typical VLA-4 antagonist can bind to VLA-4 or to a VLA-4 ligand, e.g., VCAM-1 or an extracellular matrix component, such as fibronectin or osteopontin. A VLA-4 antagonist that binds to VLA-4 may bind to either the α4 subunit or the β1 subunit, or to both. A VLA-4 antagonist may also interact with other α4 subunit containing integrins (e.g., α4β7) or with other β1 containing integrins. A VLA-4 antagonist may bind to VLA-4 or to a VLA-4 ligand with a K_{d} of less than 10⁻⁶, 10⁻⁷, 10⁻⁸, 10⁻⁹, or 10⁻¹⁰ M.

As used herein, the term "antibody" refers to a protein that includes at least one immunoglobulin variable region, e.g., an amino acid sequence that provides an immunoglobulin variable domain or immunoglobulin variable domain sequence. For example, an antibody can include a heavy (H) chain variable region (abbreviated herein as VH), and a light (L) chain variable region (abbreviated herein as VL). In another example, an antibody includes two heavy (H) chain variable regions and two light (L) chain variable regions. The term "antibody" encompasses antigen-binding fragments of antibodies (e.g., single chain antibodies, Fab fragments, F(ab')₂ fragments, Fd fragments, Fv fragments, and dAb fragments) as well as complete antibodies, e.g., intact immunoglobulins of types IgA, IgG, IgE, IgD, IgM (as well as subtypes thereof). The light chains of the immunoglobulin may be of types kappa or lambda. In one embodiment, the antibody is glycosylated. An antibody can be functional for antibody-dependent cytotoxicity and/or complement-mediated cytotoxicity, or may be non-functional for one or both of these activities.

The VH and VL regions can be further subdivided into regions of hypervariability, termed "complementarity determining regions" ("CDR"), interspersed with regions that are more conserved, termed "framework regions" (FR). The extent of the FR's and CDR's has been precisely defined (see, Kabat, E.A., et al. (1991) Sequences of Proteins of Immunological Interest, Fifth Edition, US Department of Health and Human Services, NIH Publication No. 91-3242; and Chothia, C. et al. (1987) J. Mol. Biol. 196:901-917). Kabat definitions are used herein. Each VH and VL is typically composed of three CDR's and four FR's, arranged from amino-terminus to carboxyl-terminus in the following order: FR1, CDR1, FR2, CDR2, FR3, CDR3, FR4.

An "immunoglobulin domain" refers to a domain from the variable or constant domain of immunoglobulin molecules. Immunoglobulin domains typically contain two β-sheets formed of about seven β-strands, and a conserved disulphide bond (see, e.g., A. F. Williams and A. N. Barclay 1988 Ann. Rev Immunol. 6:381-405).

As used herein, an "immunoglobulin variable domain sequence" refers to an amino acid sequence that can form the structure of an immunoglobulin variable domain. For example, the sequence may include all or part of the amino acid sequence of a naturally-occurring variable domain. For example, the sequence may omit one, two or more N- or C-terminal amino acids, internal amino acids, may include one or more insertions or additional terminal amino acids, or may include other alterations. In one embodiment, a polypeptide that includes an immunoglobulin variable domain sequence can associate with another immunoglobulin variable domain sequence to form a target binding structure (or "antigen binding site"), e.g., a structure that interacts with VLA-4.

The VH or VL chain of the antibody can further include all or part of a heavy or light chain constant region, to thereby form a heavy or light immunoglobulin chain, respectively. In one embodiment, the antibody is a tetramer of two heavy immunoglobulin chains and two light immunoglobulin chains. The heavy and light immunoglobulin chains can be connected by disulfide bonds. The heavy chain constant region typically includes three constant domains, CH1, CH2 and CH3. The light chain constant region typically includes a CL domain. The variable region of the heavy and light chains contains a binding domain that interacts with an antigen. The constant regions of the antibodies typically mediate the binding of the antibody to host tissues or factors, including various cells of the immune system (e.g., effector cells) and the first component (Clq) of the classical complement system.

One or more regions of an antibody can be human, effectively human, or humanized. For example, one or more of the variable regions can be human or effectively human. For example, one or more of the CDRs, e.g., HC CDR1, HC CDR2, HC CDR3, LC CDR1, LC CDR2, and LC CDR3, can be human. Each of the light chain CDRs can be human. HC CDR3 can be human. One or more of the framework regions can be human, e.g., FR1, FR2, FR3, and FR4 of the HC or LC. In one embodiment, all the framework regions are human, e.g., derived from a human somatic cell, e.g., a hematopoietic cell that produces immunoglobulins or a non-hematopoietic cell. In one embodiment, the human sequences are germline sequences, e.g., encoded by a germline nucleic acid. One or more of the constant regions can be human, effectively human, or humanized. In another embodiment, at least 70, 75, 80, 85, 90, 92, 95, or 98% of the framework regions (e.g., FR1, FR2, and FR3, collectively, or FR1, FR2, FR3, and FR4, collectively) or the entire antibody can be human,. effectively human, or humanized. For example, FR1, FR2, and FR3 collectively can be at least 70, 75, 80, 85, 90, 92, 95, 98, or 99% identical to a human sequence encoded by a human germline segment.

An "effectively human" immunoglobulin variable region is an immunoglobulin variable region that includes a sufficient number of human framework amino acid positions such that the immunoglobulin variable region does not elicit an immunogenic response in a normal human. An "effectively human" antibody is an antibody that includes a sufficient number of human amino acid positions such that the antibody does not elicit an immunogenic response in a normal human.

A "humanized" immunoglobulin variable region is an immunoglobulin variable region that is modified such that the modified form elicits less of an immune response in a human than does the non-modified form, e.g., is modified to include a sufficient number of human framework amino acid positions such that the immunoglobulin variable region does not elicit an immunogenic response in a normal human. Descriptions of "humanized" immunoglobulins include, for example, US Pat. No. 6,407,213 and US Pat. No. 5,693,762. In some cases, humanized immunoglobulins can include a non-human amino acid at one or more framework amino acid positions.

All or part of an antibody can be encoded by an immunoglobulin gene or a segment thereof. Exemplary human immunoglobulin genes include the kappa, lambda, alpha (IgA1 and IgA2), gamma (IgG1, IgG2, IgG3, IgG4), delta, epsilon and mu constant region genes, as well as the myriad immunoglobulin variable region genes. Full-length immunoglobulin "light chains" (about 25 Kd or 214 amino acids) are encoded by a variable region gene at the NH2-terminus (about 110 amino acids) and a kappa or lambda constant region gene at the COOH-terminus. Full-length immunoglobulin "heavy chains" (about 50 Kd or 446 amino acids), are similarly encoded by a variable region gene (about 116 amino acids) and one of the other aforementioned constant region genes, e.g., gamma (encoding about 330 amino acids).

The term "antigen-binding fragment" of a full length antibody refers to one or more fragments of a full-length antibody that retain the ability to specifically bind to a target of interest, e.g., VLA-4. Examples of binding fragments encompassed within the term "antigen-binding fragment" of a full length antibody include (i) a Fab fragment, a monovalent fragment consisting of the VL, VH, CL and CH1 domains; (ii) a F(ab')₂ fragment, a bivalent fragment including two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR) that retains functionality. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules known as single chain Fv (scFv). See, *e.g.,* Bird et al. (1988) Science 242:423-426; and Huston et al. (1988) Proc. Natl. Acad. Sci. USA 85:5879-5883.

### DETAILED DESCRIPTION

Multiple sclerosis (MS) is a central nervous system disease that is characterized by inflammation and loss of myelin sheaths. Previous studies have shown that VLA-4 expression on peripheral blood lymphocytes is downregulated after treatment of multiple sclerosis with interferon beta, suggesting that anti-VLA-4 antibodies and interferon beta act on the same pathway (Calabresi et al., Neurology 49:1111-1116, 1997). It has now been found that a VLA-4 binding protein (e.g., a blocking antibody) is dramatically effective for improving the condition of patients who previously received therapy (preferably interferon therapy) for MS, and who demonstrated an inadequate response to the therapy, or who initially demonstrated an adequate response to the therapy, which was later inadequate.

### Prior treatment

The methods described herein involve subjects that have previously received therapy or treatment, e.g., an agent, for an inflammatory disorder, e.g., MS. Preferred prior treatment agents are biologic agents, e.g., recombinant interferon beta. Nonlimiting examples of such prior treatments include the following examples:
▪ interferons, e.g., human interferon beta-1a (e.g., AVONEX® or Rebif®)) and interferon beta-1b (BETASERON™; human interferon beta substituted at position 17; Berlex/Chiron);
▪ glatiramer acetate (also termed Copolymer 1, Cop-1; COPAXONE™; Teva Pharmaceutical Industries, Inc.);
▪ fumarates, e.g., dimethyl fumarate (e.g., Fumaderm®);
▪ Rituxan® (rituximab) or another anti-CD20 antibody, e.g., one that competes with or binds an overlapping epitope with rituximab;
▪ mixtoxantrone (NOVANTRONE®, Lederle);
▪ a chemotherapeutic, e.g., clabribine (LEUSTATIN®), azathioprine (IMURAN®), cyclophosphamide (CYTOXAN®), cyclosporine-A, methotrexate, 4-aminopyridine, and tizanidine;
▪ a corticosteroid, e.g., methylprodnisolone (MEDRONE®, Pfizer), prednisone;
▪ an immunoglobulin, e.g., Rituxan® (rituximab); CTLA4 Ig; alemtuzumab (MabCAMPATH®) or daclizumab (an antibody that binds CD25);
▪ statins;
▪ immunoglobulin G i.v. (IgGIV)
▪ azathioprine;
▪ TNF antagonists.

Glatiramer acetate is protein formed of a random chain of amino acids - glutamic acid, lysine, alanine and tyrosine (hence GLATiramer). It can be synthesized in solution from these amino acids a ratio of approximately 5 parts alanine to 3 of lysine, 1.5 of glutamic acid and 1 of tyrosine using N-carboxyamino acid anhydrides.

Additional prior agents include antibodies or antagonists of other human cytokines or growth factors, for example, TNF, LT, IL- 1, IL-2, IL-6, IL-7, IL-8, IL-12 IL- 15, IL-16, IL-18, EMAP-11, GM- CSF, FGF, and PDGF. Still other exemplary second agents include antibodies to cell surface molecules such as CD2, CD3, CD4, CD8, CD25, CD28, CD30, CD40, CD45, CD69, CD80, CD86, CD90 or their ligands. For example, daclizubmab is an anti-CD25 antibody that may ameliorate multiple sclerosis.

Still other exemplary antibodies include antibodies that provide an activity of an agent described herein, e.g., an antibody that engages an interferon receptor, e.g., an interferon beta receptor.

Still other additional exemplary prior agents include: FK506, rapamycin, mycophenolate mofetil, leflunomide, non-steroidal anti-inflammatory drugs (NSAIDs), for example, phosphodiesterase inhibitors, adenosine agonists, antithrombotic agents, complement inhibitors, adrenergic agents, agents that interfere with signaling by proinflammatory cytokines as described herein, IL- Iβ converting enzyme inhibitors (e.g., Vx740), anti-P7s, PSGL, TACE inhibitors, T-cell signaling inhibitors such as kinase inhibitors, metal loproteinase inhibitors, sulfasalazine, azathloprine, 6-mercaptopurines, angiotensin converting enzyme inhibitors, soluble cytokine receptors and derivatives thereof, as described herein, anti-inflammatory cytokines (e.g. IL-4, IL-10, IL-13 and TGF).

In some embodiments, a prior agent may have been used to treat one or more symptoms or side effects of MS. Such agents include, e.g., amantadine, baclofen, papaverine, meclizine, hydroxyzine, sulfamethoxazole, ciprofloxacin, docusate, pemoline, dantrolene, desmopressin, dexamethasone, tolterodine, phenytoin, oxybutynn, bisacodyl, venlafaxine, amitriptyline, methenamine, clonazepam, isoniazid, vardenafil, nitrofurantoin, psyllium hydrophilic mucilloid, alprostadil, gabapentin, nortriptyline, paroxetine, propantheline bromide, modafinil, fluoxetine, phenazopyridine, methylprednisolone, carbamazepine, imipramine, diazepam, sildenafil, bupropion, and sertraline. Many prior agents that are small molecules have a molecular weight between 150 and 5000 Daltons.

Examples of TNF antagonists include chimeric, humanized, human or in vitro generated antibodies (or antigen-binding fragments thereof) to TNF (e.g., human TNF α), such as D2E7, (human TNFα antibody, US Pat. No. 6,258,562; BASF), CDP-571/CDP-870/BAY-10-3356 (humanized anti-TNFα antibody; Celltech/Pharmacia), cA2 (chimeric anti-TNFα antibody; REMICADE™, Centocor); anti-TNF antibody fragments (e.g., CPD870); soluble fragments of the TNF receptors, e.g., p55 or p75 human TNF receptors or derivatives thereof, e.g., 75 kdTNFR-IgG (75 kD TNF receptor-IgG fusion protein, ENBREL™; Immunex; see, e.g., Arthritis & Rheumatism (1994) Vol. 37, S295; J. Invest. Med. (1996) Vol. 44, 235A), p55 kdTNFR-IgG (55 kD TNF receptor-IgG fusion protein (LENERCEPT™)); enzyme antagonists, e.g., TNFα converting enzyme (TACE) inhibitors (e.g., an alpha-sulfonyl hydroxamic acid derivative, WO 01/55112, and N-hydroxyformamide TACE inhibitor GW 3333, -005, or -022); and TNF-bp/s-TNFR (soluble TNF binding protein; see, e.g., Arthritis & Rheumatism (1996) Vol. 39, No. 9 (supplement), S284; Amer. J. Physiol. - Heart and Circulatory Physiology (1995) Vol. 268, pp. 37-42).

The safety and efficacy profile of VLA-4 blocking therapy (e.g., administration of a VLA-4 binding antibody such as natalizumab) in patients having an inadequate response to interferon beta therapy shows that VLA-4 blocking therapy will be useful in treating patients having an inadequate response to other therapies, e.g., other biologic therapies, as well.

### Natalizumab and Other VLA-4 Binding Antibodies

Natalizumab, an α4 integrin binding antibody, inhibits the migration of leukocytes from the blood to the central nervous system. Natalizumab binds to VLA-4 on the surface of activated T-cells and other mononuclear leukocytes. It can disrupt adhesion between the T-cell and endothelial cells, and thus prevent migration of mononuclear leukocytes across the endothelium and into the parenchyma. As a result, the levels of proinflammatory cytokines can also be reduced.

Natalizumab can decrease the number of brain lesions and clinical relapses in patients with relapse remitting multiple sclerosis and relapsing secondary-progressive multiple sclerosis. The results described herein show that natalizumab can be safely administered to patients with multiple sclerosis who have previously received interferon, e.g., interferon beta(e.g., IFN-beta-1a) therapy. Natalizumab shows unexpected efficacy in patients who have had an inadequate response to interferon beta therapy.

Natalizumab and related VLA-4 binding antibodies are described, e.g., in US Pat. No. 5,840,299. Monoclonal antibodies 21.6 and HP1/2 are exemplary murine monoclonal antibodies that bind VLA-4. Natalizumab is a humanized version of murine monoclonal antibody 21.6 (see, e.g., US Pat No. 5,840,299). A humanized version of HP1/2 has also been described (see, e.g., US Pat. No. 6,602,503). Several additional VLA-4 binding monoclonal antibodies, such as HP2/1, HP2/4, L25 and P4C2, are described, e.g., in US Pat. No. 6,602,503; Sanchez-Madrid et al., 1986 Eur. J. Immunol., 16:1343-1349; Hemler et al., 1987 J. Biol. Chem. 2:11478-11485; Issekutz and Wykretowicz, 1991, J. Immunol., 147: 109 (TA-2 mab); Pulido et al., 1991 J. Biol. Chem., 266(16): 10241-10245; and US Pat. No. 5,888,507.

Some VLA-4 binding antibodies recognize epitopes of the α4 subunit that are involved in binding to a cognate ligand, e.g., VCAM-1 or fibronectin. Many such antibodies inhibit binding of VLA-4 to cognate ligands (e.g., VCAM-1 and fibronectin).

Some useful VLA-4 binding antibodies can interact with VLA-4 on cells, e.g., lymphocytes, but do not cause cell aggregation. However, other VLA-4 binding antibodies have been observed to cause such aggregation. HP1/2 does not cause cell aggregation. The HP1/2 monoclonal antibody (Sanchez-Madrid et al., 1986) has an extremely high potency, blocks VLA-4 interaction with both VCAM1 and fibronectin, and has the specificity for epitope B on VLA-4. This antibody and other B epitope-specific antibodies (such as B1 or B2 epitope binding antibodies; Pulido et al., 1991, supra) represent one class of VLA-4 binding antibodies that can be used in the methods described herein. Antibodies that compete for binding with a VLA-4 binding antibody, e.g., natalizumab, can also be used in the methods described herein.

An exemplary VLA-4 binding antibody has one or more CDRs, e.g., all three HC CDRs and/or all three LC CDRs of a particular antibody disclosed herein, or CDRs that are, in sum, at least 80, 85, 90, 92, 94, 95, 96, 97, 98, 99% identical to such an antibody, e.g., natalizumab. In one embodiment, the H1 and H2 hypervariable loops have the same canonical structure as those of an antibody described herein. In one embodiment, the L1 and L2 hypervariable loops have the same canonical structure as those of an antibody described herein.

In one embodiment, the amino acid sequence of the HC and/or LC variable domain sequence is at least 70, 80, 85, 90, 92, 95, 97, 98, 99, or 100% identical to the amino acid sequence of the HC and/or LC variable domain of an antibody described herein, e.g., natalizumab. The amino acid sequence of the HC and/or LC variable domain sequence can differ by at least one amino acid, but no more than ten, eight, six, five, four, three, or two amino acids from the corresponding sequence of an antibody described herein, e.g., natalizumab. For example, the differences may be primarily or entirely in the framework regions.

The amino acid sequences of the HC and LC variable domain sequences can be encoded by a nucleic acid sequence that hybridizes under high stringency conditions to a nucleic acid sequence described herein or one that encodes a variable domain or an amino acid sequence described herein. In one embodiment, the amino acid sequences of one or more framework regions (e.g., FR1, FR2, FR3, and/or FR4) of the HC and/or LC variable domain are at least 70, 80, 85, 90, 92, 95, 97, 98, 99, or 100% identical to corresponding framework regions of the HC and LC variable domains of an antibody described herein. In one embodiment, one or more heavy or light chain framework regions (e.g., HC FR1, FR2, and FR3) are at least 70, 80, 85, 90, 95, 96, 97, 98, or 100% identical to the sequence of corresponding framework regions from a human germline antibody.

Calculations of "homology" or "sequence identity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes). The optimal alignment is determined as the best score using the GAP program in the GCG software package with a Blossum 62 scoring matrix with a gap penalty of 12, a gap extend penalty of 4, and a frameshift gap penalty of 5. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "identity" is equivalent to amino acid or nucleic acid "homology"). The percent identity between the two sequences is a function of the number of identical positions shared by the sequences.

As used herein, the term "hybridizes under high stringency conditions" describes conditions for hybridization and washing. Guidance for performing hybridization reactions can be found in Current Protocols in Molecular Biology, John Wiley & Sons, N.Y. (1989), 6.3.1-6.3.6, which is incorporated by reference. Aqueous and nonaqueous methods are described in that reference and either can be used. High stringency hybridization conditions include hybridization in 6X SSC at about 45°C, followed by one or more washes in 0.2X SSC, 0.1% SDS at 65°C, or substantially similar conditions.

### Antibody Generation

Antibodies that bind to VLA-4 can be generated by immunization, e.g., using an animal, or by in vitro methods such as phage display. All or part of VLA-4 can be used as an imnunogen. For example, the extracellular region of the α4 subunit can be used as an immunogen. In one embodiment, the immunized animal contains immunoglobulin producing cells with natural, human, or partially human immunoglobulin loci. In one embodiment, the non-human animal includes at least a part of a human immunoglobulin gene. For example, it is possible to engineer mouse strains deficient in mouse antibody production with large fragments of the human Ig loci. Using the hybridoma technology, antigen-specific monoclonal antibodies derived from the genes with the desired specificity may be produced and selected. See, e.g., XenoMouse™, Green et al., Nature Genetics 7:13-21 (1994), US 2003-0070185, US Pat. No. 5,789,650, and WO 96/34096.

Non-human antibodies to VLA-4 can also be produced, e.g., in a rodent. The non-human antibody can be humanized, e.g., as described in US Pat. No. 6,602,503, EP 239 400, US Pat. No. 5,693,761, and US Pat. No. 6,407,213.

EP 239 400 (Winter et al.) describes altering antibodies by substitution (within a given variable region) of their complementarity determining regions (CDRs) for one species with those from another. CDR-substituted antibodies can be less likely to elicit an immune response in humans compared to true chimeric antibodies because the CDR-substituted antibodies contain considerably less non-human components (Riechmann et al., 1988, Nature 332, 323-327; Verhoeyen et al., 1988, Science 239, 1534-1536). Typically, CDRs of a murine antibody substituted into the corresponding regions in a human antibody by using recombinant nucleic acid technology to produce sequences encoding the desired substituted antibody. Human constant region gene segments of the desired isotype (usually gamma I for CH and kappa for CL) can be added and the humanized heavy and light chain genes can be co-expressed in mammalian cells to produce soluble humanized antibody.

Queen et al. (Proc. Natl. Acad. Sci. U. S. A. 86:10029-33, 1989) and WO 90/07861 have described a process that includes choosing human V framework regions by computer analysis for optimal protein sequence homology to the V region framework of the original murine antibody, and modeling the tertiary structure of the murine V region to visualize framework amino acid residues that are likely to interact with the murine CDRs. These murine amino acid residues are then superimposed on the homologous human framework. See also US Pat. Nos. 5,693,762; 5,693,761; 5,585,089; and 5,530,101. Tempest et al., 1991, Biotechnology 9:266-271, utilize, as standard, the V region frameworks derived from NEWM and REI heavy and light chains, respectively, for CDR-grafting without radical introduction of mouse residues. An advantage of using the Tempest et al. approach to construct NEWM and REI based humanized antibodies is that the three dimensional structures of NEWM and REI variable regions are known from x-ray crystallography and thus specific interactions between CDRs and V region framework residues can be modeled.

Non-human antibodies can be modified to include substitutions that insert human immunoglobulin sequences, e.g., consensus human amino acid residues at particular positions, e.g., at one or more (preferably at least five, ten, twelve, or all) of the following positions: (in the FR of the variable domain of the light chain) 4L, 35L, 36L, 38L, 43L, 44L, 58L, 46L, 62L, 63L, 64L, 65L, 66L, 67L, 68L, 69L, 70L, 71L, 73L, 85L, 87L, 98L, and/or (in the FR of the variable domain of the heavy chain) 2H, 4H, 24H, 36H, 37H, 39H, 43H, 45H, 49H, 58H, 60H, 67H, 68H, 69H, 70H, 73H, 74H, 75H, 78H, 91H, 92H, 93H, and/or 103H (according to the Kabat numbering). See, e.g., US Pat. No. 6,407,213.

Fully human monoclonal antibodies that bind to VLA-4 can be produced, e.g., using in vitro-primed human splenocytes, as described by Boerner et al., 1991, J. Immunol., 147, 86-95. They may be prepared by repertoire cloning as described by Persson et al., 1991, Proc. Nat. Acad. Sci. USA, 88: 2432-2436 or by Huang and Stollar, 1991, J. Immunol. Methods 141,227-236; also US Pat. No. 5,798,230. Large nonimmunized human phage display libraries may also be used to isolate high affinity antibodies that can be developed as human therapeutics using standard phage technology (see, e.g., Vaughan et al, 1996; Hoogenboom et al. (1998) Immunotechnology 4:1-20; and Hoogenboom et al (2000) Immunol Today 2:371-8; US 2003-0232333).

### Antibody Production

Antibodies can be produced in prokaryotic and eukaryotic cells. In one embodiment, the antibodies (e.g., scFv's) are expressed in a yeast cell such as *Pichia* (see, e.g., Powers et al. (2001) J Immunol Methods. 251:123-35), *Hanseula,* or *Saccharomyces*.

In one embodiment, antibodies, particularly full length antibodies, e.g., IgG's, are produced in mammalian cells. Exemplary mammalian host cells for recombinant expression include Chinese Hamster Ovary (CHO cells) (including dhfr- CHO cells, described in Urlaub and Chasin (1980) Proc. Natl. Acad. Sci. USA 77:4216-4220, used with a DHFR selectable marker, e.g., as described in Kaufman and Sharp (1982) Mol. Biol. 159:601-621), lymphocytic cell lines, e.g., NS0 myeloma cells and SP2 cells, COS cells, K562, and a cell from a transgenic animal, e.g., a transgenic mammal. For example, the cell is a mammary epithelial cell.

In addition to the nucleic acid sequence encoding the immunoglobulin domain, the recombinant expression vectors may carry additional nucleic acid sequences, such as sequences that regulate replication of the vector in host cells *(e.g.,* origins of replication) and selectable marker genes. The selectable marker gene facilitates selection of host cells into which the vector has been introduced (see, e.g., US Pat. Nos. 4,399,216,4,634,665 and 5,179,017). Exemplary selectable marker genes include the dihydrofolate reductase (DHFR) gene (for use in *dhfr⁻* host cells with methotrexate selection/amplification) and the *neo* gene (for G418 selection).

In an exemplary system for recombinant expression of an antibody (e.g., a full length antibody or an antigen-binding portion thereof), a recombinant expression vector encoding both the antibody heavy chain and the antibody light chain is introduced into *dhfr-* CHO cells by calcium phosphate-mediated transfection. Within the recombinant expression vector, the antibody heavy and light chain genes are each operatively linked to enhancer/promoter regulatory elements (e.g., derived from SV40, CMV, adenovirus and the like, such as a CMV enhancer/AdMLP promoter regulatory element or an SV40 enhancer/AdMLP promoter regulatory element) to drive high levels of transcription of the genes. The recombinant expression vector also carries a DHFR gene, which allows for selection of CHO cells that have been transfected with the vector using methotrexate selection/amplification. The selected transformant host cells are cultured to allow for expression of the antibody heavy and light chains and intact antibody is recovered from the culture medium. Standard molecular biology techniques are used to prepare the recombinant expression vector, to transfect the host cells, to select for transformants, to culture the host cells, and to recover the antibody from the culture medium. For example, some antibodies can be isolated by affinity chromatography with a Protein A or Protein G.

Antibodies may also include modifications, e.g., modifications that alter Fc function, e.g., to decrease or remove interaction with an Fc receptor or with C1q, or both. For example, the human IgG1 constant region can be mutated at one or more residues, e.g., one or more of residues 234 and 237, e.g., according to the numbering in US Pat. No. 5,648,260. Other exemplary modifications include those described in US Pat. No. 5,648,260.

For some antibodies that include an Fc domain, the antibody production system may be designed to synthesize antibodies in which the Fc region is glycosylated. For example, the Fc domain of IgG molecules is glycosylated at asparagine 297 in the CH2 domain. This asparagine is the site for modification with biantennary-type oligosaccharides. This glycosylation participates in effector functions mediated by Fcγ receptors and complement C1q (Burton and Woof (1992) Adv. Immunol. 51:1-84; Jefferis et al. (1998) Immunol. Rev. 163:59-76). The Fc domain can be produced in a mammalian expression system that appropriately glycosylates the residue corresponding to asparagine 297. The Fc domain can also include other eukaryotic post-translational modifications.

Antibodies can also be produced by a transgenic animal. For example, US Pat. No. 5,849,992 describes a method for expressing an antibody in the mammary gland of a transgenic mammal. A transgene is constructed that includes a milk-specific promoter and nucleic acid sequences encoding the antibody of interest, e.g., an antibody described herein, and a signal sequence for secretion. The milk produced by females of such transgenic mammals includes, secreted-therein, the antibody of interest, e.g., an antibody described herein. The antibody can be purified from the milk, or for some applications, used directly.

Antibodies can be modified, e.g., with a moiety that improves its stabilization and/or retention in circulation, e.g., in blood, serum, lymph, bronchoalveolar lavage, or other tissues, e.g., by at least 1.5,2, 5, 10, or 50 fold.

For example, a VLA-4 binding antibody can be associated with a polymer, e.g., a substantially non-antigenic polymer, such as polyalkylene oxide or a polyethylene oxide. Suitable polymers will vary substantially by weight. Polymers having molecular number average weights ranging from about 200 to about 35,000 daltons (or about 1,000 to about 15,000, and 2,000 to about 12,500) can be used.

For example, a VLA-4 binding antibody can be conjugated to a water soluble polymer, e.g., a hydrophilic polyvinyl polymer, e.g. polyvinylalcohol or polyvinylpyrrolidone. A non-limiting list of such polymers include polyalkylene oxide homopolymers such as polyethylene glycol (PEG) or polypropylene glycols, polyoxyethylenated polyols, copolymers thereof and block copolymers thereof, provided that the water solubility of the block copolymers is maintained. Additional useful polymers include polyoxyalkylenes such as polyoxyethylene, polyoxypropylene, and block copolymers of polyoxyethylene and polyoxypropylene (Pluronics); polymethacrylates; carbomers; branched or unbranched polysaccharides that comprise the saccharide monomers D-mannose, D- and L-galactose, fucose, fructose, D-xylose, L-arabinose, D-glucuronic acid, sialic acid, D-galacturonic acid, D-mannuronic acid (e.g. polymannuronic acid, or alginic acid), D-glucosamine, D-galactosamine, D-glucose and neuraminic acid including homopolysaccharides and heteropolysaccharides such as lactose, amylopectin, starch, hydroxyethyl starch, amylose, dextrane sulfate, dextran, dextrins, glycogen, or the polysaccharide subunit of acid mucopolysaccharides, e.g. hyaluronic acid; polymers of sugar alcohols such as polysorbitol and polymannitol; heparin or heparon.

### Pharmaceutical Compositions

A VLA-4 binding agent, such as a VLA-4 binding antibody, (e.g., natalizumab) can be formulated as a pharmaceutical composition. Typically, a pharmaceutical composition includes a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible.

A "pharmaceutically acceptable salt" refers to a salt that retains the desired biological activity of the parent compound and does not impart any undesired toxicological effects (see, *e.g.,* Berge, S.M., et al. (1977) J. Pharm. Sci. 66:1-19). Examples of such salts include acid addition salts and base addition salts. Acid addition salts include those derived from nontoxic inorganic acids, such as hydrochloric, nitric, phosphoric, sulfuric, hydrobromic, hydroiodic, and the like, as well as from nontoxic organic acids such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

Natalizumab and other agents described herein can be formulated according to standard methods. Pharmaceutical formulation is a well-established art, and is further described in Gennaro (ed.), Remington: The Science and Practice of Pharmacy, 20th ed., Lippincott, Williams & Wilkins (2000) (ISBN: 0683306472); Ansel et al., Pharmaceutical Dosage Forms and Drug Delivery Systems, 7th Ed.., Lippincott Williams & Wilkins Publishers (1999) (ISBN: 0683305727); and Kibbe (ed.), Handbook of Pharmaceutical Excipients American Pharmaceutical Association, 3rd ed. (2000) (ISBN: 091733096X).

In one embodiment, natalizumab or another agent (e.g., another antibody) can be formulated with excipient materials, such as sodium chloride, sodium dibasic phosphate heptahydrate, sodium monobasic phosphate, and polysorbate 80. It can be provided, for example, in a buffered solution at a concentration of about 20 mg/ml and can be stored at 2-8°C. Natalizumab can be formulated as described on the manufacturer's label.

Pharmaceutical compositions may also be in a variety of other forms. These include, for example, liquid, semi-solid and solid dosage forms, such as liquid solutions (e.g., injectable and infusible solutions), dispersions or suspensions, tablets, pills, powders, liposomes and suppositories. The preferred form can depend on the intended mode of administration and therapeutic application. Typically compositions for the agents described herein are in the form of injectable or infusible solutions.

Such compositions can be administered by a parenteral mode (*e.g.,* intravenous, subcutaneous, intraperitoneal, or intramuscular injection). The phrases "parenteral administration" and "administered parenterally" as used herein mean modes of administration other than enteral and topical administration, usually by injection, and include, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion.

Pharmaceutical compositions typically must be sterile and stable under the conditions of manufacture and storage. A pharmaceutical composition can also be tested to insure it meets regulatory and industry standards for administration.

The composition can be formulated as a solution, microemulsion, dispersion, liposome, or other ordered structure suitable to high drug concentration. Sterile injectable solutions can be prepared by incorporating an agent described herein in the required amount in an appropriate solvent with one or a combination of ingredients enumerated above, as required, followed by filtered sterilization. Generally, dispersions are prepared by incorporating an agent described herein into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of an agent described herein plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

### Administration

A VLA-4 binding antibody can be administered to a subject, e.g., a human subject, by a variety of methods. For many applications, the route of administration is one of: intravenous injection or infusion, subcutaneous injection, or intramuscular injection. A VLA-4 binding antibody, such as natalizumab, can be administered as a fixed dose, or in a mg/kg dose, but preferably as a fixed dose. The antibody can be administered intravenously (IV) or subcutaneously (SC).

The antibody, e.g., natalizumab, is typically administered at a fixed unit dose of between 50-1000 mg IV, e.g., between 100-600 mg IV, e.g., about 300 mg IV. A unit dose can be administered every 4 weeks or less or more frequently, e.g., every 2 weeks or weekly. When administered subcutaneously, the antibody is typically administered at a dose between 50-100 mg SC (e.g., 75 mg), e.g., at least once a week (e.g., twice a week). It can also be administered in a bolus at a dose of between 1 and 10 mg/kg, e.g., about 6.0, 4.0, 3.0, 2.0, 1.0 mg/kg. In some cases, continuous administration may be indicated, e.g., via a subcutaneous pump.

The dose can also be chosen to reduce or avoid production of antibodies against the VLA-4 binding antibody, to achieve greater than 40, 50, 70, 75, or 80% saturation of the α4 subunit, to achieve to less than 80, 70, 60, 50, or 40% saturation of the α4 subunit, or to prevent an increase the level of circulating white blood cells

In certain embodiments, the active agent may be prepared with a carrier that will protect the compound against rapid release, such as a controlled release formulation, including implants, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known. *See, e.g.,* Sustained and Controlled Release Drug Delivery Systems, J.R. Robinson, ed., Marcel Dekker, Inc., New York, 1978.

Pharmaceutical compositions can be administered with medical devices. For example, pharmaceutical compositions can be administered with a needleless hypodermic injection device, such as the devices disclosed in US Pat. Nos. 5,399,163, 5,383,851, 5,312,335, 5,064,413, 4,941,880, 4,790,824, or 4,596,556. Examples of well-known implants and modules include: US Pat. No. 4,487,603, which discloses an implantable micro-infusion pump for dispensing medication at a controlled rate; US Pat. No. 4,486,194, which discloses a therapeutic device for administeringmedicants through the skin; US Pat. No. 4,447,233, which discloses a medication infusion pump for delivering medication at a precise infusion rate; US Pat. No. 4,447,224, which discloses a variable flow implantable infusion apparatus for continuous drug delivery; US Pat. No. 4,439,196, which discloses an osmotic drug delivery system having multichamber compartments; and US Pat. No. 4,475,196, which discloses an osmotic drug delivery system. Of course, many other such implants, delivery systems, and modules are also known.

Dosage unit form or "fixed dose" as used herein refers to physically discrete units suited as unitary dosages for the subjects to be treated; each unit contains a predetermined quantity of active compound calculated to produce the desired therapeutic effect in association with the required pharmaceutical carrier and optionally in association with the other agent.

A pharmaceutical composition may include a "therapeutically effective amount" of an agent described herein. A therapeutically effective amount of an agent may also vary according to factors such as the disease state, age, sex, and weight of the individual, and the ability of the compound to elicit a desired response in the individual, e.g., amelioration of at least one disorder parameter, e.g., a multiple sclerosis parameter, or amelioration of at least one symptom of the disorder, e.g., multiple sclerosis. A therapeutically effective amount is also one in which any toxic or detrimental effects of the composition is outweighed by the therapeutically beneficial effects.

### Multiple Sclerosis

Multiple sclerosis (MS) is a central nervous system disease that is characterized by inflammation and loss of myelin sheaths.

Patients having MS may be identified by criteria establishing a diagnosis of clinically definite MS as defined by the workshop on the diagnosis of MS (Poser et al., Ann. Neurol. 13:227, 1983). Briefly, an individual with clinically definite MS has had two attacks and clinical evidence of either two lesions or clinical evidence of one lesion and paraclinical evidence of another, separate lesion. Definite MS may also be diagnosed by evidence of two attacks and oligoclonal bands of IgG in cerebrospinal fluid or by combination of an attack, clinical evidence of two lesions and oligoclonal band of IgG in cerebrospinal fluid. The McDonald criteria can also be used to diagnose MS. (McDonald et al., 2001, Recommended diagnostic criteria for multiple sclerosis: guidelines from the International Panel on the Diagnosis of Multiple Sclerosis, Ann Neurol 50:121-127). The McDonald criteria include the use of MRI evidence of CNS impairment over time to be used in diagnosis of MS, in the absence of multiple clinical attacks. Effective treatment of multiple sclerosis may be evaluated in several different ways. The following parameters can be used to gauge effectiveness of treatment. Two exemplary criteria include: EDSS (extended disability status scale), and appearance of exacerbations on MRI (magnetic resonance imaging). The EDSS is a means to grade clinical impairment due to MS (Kurtzke, Neurology 33:1444, 1983). Eight functional systems are evaluated for the type and severity of neurologic impairment. Briefly, prior to treatment, patients are evaluated for impairment in the following systems: pyramidal, cerebella, brainstem, sensory, bowel and bladder, visual, cerebral, and other. Follow-ups are conducted at defined intervals. The scale ranges from 0 (normal) to 10 (death due to MS). A decrease of one full step indicates an effective treatment (Kurtzke, Ann. Neurol. 36:573-79, 1994).

Exacerbations are defined as the appearance of a new symptom that is attributable to MS and accompanied by an appropriate new neurologic abnormality (IFNB MS Study Group, supra). In addition, the exacerbation must last at least 24 hours and be preceded by stability or improvement for at least 30 days. Briefly, patients are given a standard neurological examination by clinicians. Exacerbations are either mild, moderate, or severe according to changes in a Neurological Rating Scale (Sipe et al., Neurology 34:1368, 1984). An annual exacerbation rate and proportion of exacerbation-free patients are determined.

Therapy can be deemed to be effective if there is a statistically significant difference in the rate or proportion of exacerbation-free or relapse-free patients between the treated group and the placebo group for either of these measurements. In addition, time to first exacerbation and exacerbation duration and severity may also be measured. A measure of effectiveness as therapy in this regard is a statistically significant difference in the time to first exacerbation or duration and severity in the treated group compared to control group. An exacerbation-free or relapse-free period of greater than one year, 18 months, or 20 months is particularly noteworthy.

Clinical measurements include the relapse rate in one and two-year intervals, and a change in EDSS, including time to progression from baseline of 1.0 unit on the EDSS that persists for six months. On a Kaplan-Meier curve, a delay in sustained progression of disability shows efficacy. Other criteria include a change in area and volume of T2 images on MRI, and the number and volume of lesions determined by gadolinium enhanced images.

MRI can be used to measure active lesions using gadolinium-DTPA-enhanced imaging (McDonald et al., Ann. NeuroL 36:14, 1994) or the location and extent of lesions using T₂ -weighted techniques. Briefly, baseline MRIs are obtained. The same imaging plane and patient position are used for each subsequent study. Positioning and imaging sequences can be chosen to maximize lesion detection and facilitate lesion tracing. The same positioning and imaging sequences can be used on subsequent studies. The presence, location and extent of MS lesions can be determined by radiologists. Areas of lesions can be outlined and summed slice by slice for total lesion area. Three analyses may be done: evidence of new lesions, rate of appearance of active lesions, percentage change in lesion area (Paty et al., Neurology 43:665, 1993). Improvement due to therapy can be established by a statistically significant improvement in an individual patient compared to baseline or in a treated group versus a placebo group.

Exemplary symptoms associated with multiple sclerosis, which can be treated with the methods described herein, include: optic neuritis, diplopia, nystagmus, ocular dysmetria, internuclear ophthalmoplegia, movement and sound phosphenes, afferent pupillary defect, paresis, monoparesis, paraparesis, hemiparesis, quadraparesis, plegia, paraplegia, hemiplegia, tetraplegia, quadraplegia, spasticity, dysarthria, muscle atrophy, spasms, cramps, hypotonia, clonus, myoclonus, myokymia, restless leg syndrome, footdrop, dysfunctional reflexes, paraesthesia, anaesthesia, neuralgia, neuropathic and neurogenic pain, l'hermitte's, proprioceptive dysfunction, trigeminal neuralgia, ataxia, intention tremor, dysmetria, vestibular ataxia, vertigo, speech ataxia, dystonia, dysdiadochokinesia, frequent micturation, bladder spasticity, flaccid bladder, detrusor-sphincter dyssynergia, erectile dysfunction, anorgasmy, frigidity, constipation, fecal urgency, fecal incontinence, depression, cognitive dysfunction, dementia, mood swings, emotional lability, euphoria, bipolar syndrome, anxiety, aphasia, dysphasia, fatigue, uhthoffs symptom, gastroesophageal reflux, and sleeping disorders.

Each case of MS displays one of several patterns of presentation and subsequent course. Most commonly, MS first manifests itself as a series of attacks followed by complete or partial remissions as symptoms mysteriously lessen, only to return later after a period of stability. This is called relapsing-remitting (RR) MS. Primary-progressive (PP) MS is characterized by a gradual clinical decline with no distinct remissions, although there may be temporary plateaus or minor relief from symptoms. Secondary-progressive (SP) MS begins with a relapsing-remitting course followed by a later primary-progressive course. Rarely, patients may have a progressive-relapsing (PR) course in which the disease takes a progressive path punctuated by acute attacks. PP, SP, and PR are sometimes lumped together and called chronic progressive MS.

A few patients experience malignant MS, defined as a swift and relentless decline resulting in significant disability or even death shortly after disease onset. This decline may be arrested or decelerated by administration of a combination therapy described herein.

In addition to or prior to human studies, an animal model can be used to evaluate the efficacy of treatment. An exemplary animal model for multiple sclerosis is the experimental autoimmune encephalitis (EAE) mouse model, e.g., as described in Tuohy et al. (J. Immunol. (1988) 141:1126-1130), Sobel et al. (J. Immunol. (1984) 132: 2393-2401), and Traugott (Cell Immunol. (1989) 119: 114-129. Mice can be administered an agent described herein prior to EAE induction. Then the mice are evaluated for characteristic criteria to determine the efficacy of using the agent in the model.

### Other Disorders

The methods described herein can also be used to treat other inflammatory, immune, or autoimmune disorders in patients in which an inadequate response to an interferon therapy has been exhibited. Such disorders include, e.g., inflammation of the central nervous system (e.g., in addition to multiple sclerosis, meningitis, neuromyelitis optica, neurosarcoidosis, CNS vasculitis, encephalitis, and transverse myelitis); tissue or organ graft rejection or graft-versus-host disease; acute CNS injury, e.g., stroke or spinal cord injury; chronic renal disease; allergy, e.g., allergic asthma; type 1 diabetes; inflammatory bowel disorders, e.g., Crohn's disease, ulcerative colitis; myasthenia gravis; fibromyalgia; arthritic disorders, e.g., rheumatoid arthritis, psoriatic arthritis; inflammatory/immune skin disorders, e.g., psoriasis, vitiligo, dermatitis, lichen planus; systemic lupus erythematosus; Sjogren's Syndrome; hematological cancers, e.g., multiple myeloma, leukemia, lymphoma; solid cancers, e.g., sarcomas or carcinomas, e.g., of the lung, breast, prostate, brain; and fibrotic disorders, e.g., pulmonary fibrosis, myelofibrosis, liver cirrhosis, mesangial proliferative glomerulonephritis, crescentic glomerulonephritis, diabetic nephropathy, and renal interstitial fibrosis.

For example, a VLA-4 binding agent (e.g., a VLA-4 binding antibody, e.g., natalizumab) can be administered to treat these and other inflammatory, immune, or autoimmune disorders, e.g., disorders that exhibit an inadequate response to treatment with another agent or agents, e.g., an agent or agents described herein.

### Kits

A VLA-4 binding agent (e.g., natalizumab) can be provided in a kit. In one embodiment, the kit includes (a) a container that contains a composition that includes both VLA-4 binding agent (e.g., natalizumab) and, optionally (b) informational material. The informational material can be descriptive, instructional, marketing or other material that relates to the methods described herein and/or the use of the agents for therapeutic benefit.

The informational material of the kits is not limited in its form. In one embodiment, the informational material can include information about production of the compound, molecular weight of the compound, concentration, date of expiration, batch or production site information, and so forth. In one embodiment, the informational material relates to methods of administering the VLA-4 binding agent, e.g., in a suitable amount, manner, or mode of administration (e.g., a dose, dosage form, or mode of administration described herein). The method can be a method of treating multiple sclerosis, e.g., in a patient who has previously received interferon beta therapy.

The informational material of the kits is not limited in its form. In many cases, the informational material, e.g., instructions, is provided in printed matter, e.g., a printed text, drawing, and/or photograph, e.g., a label or printed sheet. However, the informational material can also be provided in other formats, such as Braille, computer readable material, video recording, or audio recording. In another embodiment, the informational material of the kit is contact information, e.g., a physical address, email address, website, or telephone number, where a user of the kit can obtain substantive information about agents therein and/or its use in the methods described herein. Of course, the informational material can also be provided in any combination of formats.

In addition to the VLA-4 binding agent, the composition of the kit can include other ingredients, such as a solvent or buffer, a stabilizer, or a preservative. The kit may also include other agents, e.g., a second or third agent, e.g., other therapeutic agents.

The agents can be provided in any form, e.g., liquid, dried or lyophilized form. It is preferred that the agents are substantially pure (although they can be combined together or delivered separate from one another) and/or sterile. When the agents are provided in a liquid solution, the liquid solution preferably is an aqueous solution, with a sterile aqueous solution being preferred. When the agents are provided as a dried form, reconstitution generally is by the addition of a suitable solvent. The solvent, e.g., sterile water or buffer, can optionally be provided in the kit.

The kit can include one or more containers for the composition or compositions containing the agents. In some embodiments, the kit contains separate containers, dividers or compartments for the composition and informational material. For example, the composition can be contained in a bottle, vial, or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (e.g., a pack) of individual containers, each containing one or more unit dosage forms (e.g., a dosage form described herein) of the agents. The containers can include a unit dosage, e.g., a unit that includes the VLA-4 binding agent. For example, the kit includes a plurality of syringes, ampules, foil packets, blister packs, or medical devices, e.g., each containing a unit dose. The containers of the kits can be air tight, waterproof (e.g., impermeable to changes in moisture or evaporation), and/or light-tight.

The kit optionally includes a device suitable for administration of the composition, e.g., a syringe or other suitable delivery device. The device can be provided pre-loaded with a VLA-4 binding agent, e. g., in a unit dose, or can be empty, but suitable for loading.

All references and publications included herein are incorporated herein by reference. The following example is not intended to be limiting.

### EXAMPLES

### Example 1 - AFFIRM and SENTINEL studies

The 13 month clinical endpoints of the AFFIRM and SENTINEL studies (Rudick et al., 2003, Neurology 60 Supp.1: A479) are shown in Tables 1 and 2, respectively.

**Table 1**

| | Natalizumab | Placebo |
|---|---|---|
| | N=627 | N=315 |
| Annualized relapse rate | 0.25 | 0.74 |
| Relative reduction | 66% | |
| % patients remaining relapse free | 76% | 53% |

**Table 2**

| | Natalizumab + Avonex | Placebo+ Avonex |
|---|---|---|
| | N=589 | N=582 |
| Annualized relapse rate | 0.36 | 0.78 |
| Relative reduction | 54% | |
| % patients remaining relapse free | 67% | 46% |

Other embodiments are within the scope of the following claims.

Preferred embodiments of the present invention are described below and are referred to as embodiments E1 to E20.
E 1. A method of treating a subject who has an inflammatory disorder and who exhibits an inadequate response to a first agent, the method comprising administering to the subject a VLA-4 binding protein in an amount and for a time sufficient to treat said disorder, wherein the VLA-4 binding protein is administered at least once when the first agent is not present at a therapeutic level in the subject
E 2. The method of E 1, wherein the inadequate response comprises less than a predetermined level of response.
E 3. The method of E 1, wherein the inadequate response comprises an adverse reaction or intolerable toxic response to the first agent.
E 4. The method of E1, wherein the inflammatory disorder is refractory to treatment with the first agent in the subject
E 5. The method of E 1, wherein the first agent is selected from the group consisting of an interferon, glatiramer acetate, a fumarate, mixtoxantrone, a chemotherapeutic, a corticosteroid, an immunoglobulin, a statin, azathioprine, and a TNF antagonist.
E 6. The method of E 1, wherein the subject has been administered the first agent for at least 6 months.
E 7. The method of E 1, wherein the administration of the VLA-4 binding protein is continued, in the absence of administration of the first agent, for at least 2 months.
E 8. The method of E 1, wherein the subject has not been administered the first agent for at least 3 months prior to a first administration of the VLA-4 binding protein.
E 9. The method of E 1, wherein the subject has been administered the first agent within 2 weeks prior to a first administration of the VLA-4 binding protein.
E 10. The method of E 1, further comprising monitoring the subject for an improvement in EDSS score.
E 11. The method of E 1, wherein the subject has not previously been administered a VLA-4 binding protein since being diagnosed with multiple sclerosis.
E 12. The method of E 1, wherein the VLA-4 binding protein is a VLA-4 binding antibody.
E 13, The method of E 12, wherein the VLA-4 binding antibody inhibits VLA-4 interaction with VCAM-1.
E 14. The method of E 13, wherein VLA-4 binding antibody binds at least the α chain of VLA-4.
E 15. The method of E 12, wherein the VLA-4 binding antibody is natalizumab,
E 16. The method of E. 12, wherein the VLA-4 binding antibody competes with HP 1/2 or natalizumab for binding to VLA-4.
E 17. The method of E 12, wherein the VLA-4 binding antibody is human or humanized.
E 18. The method of E 1, wherein the subject has chronic progressive multiple sclerosis, primary-progressive (PP) multiple sclerosis, secondary progressive multiple sclerosis or progressive relapsing multiple sclerosis.
E 19. The method of E1, wherein the subject is administered a plurality of doses of the VLA-4 binding protein intravenously, each dose being between 200-400 mg.
E20. A method of treating a subject who has MS and who, while being treated with interferon beta for at least 3 months, has had one or more of: (a) at least one relapse, (b) appearance of new MRI enhancing lesions, and (c) progression of disability (EDSS), the method comprising administering to the subject a VLA-4 blocking antibody, or VLA-4 binding fragment thereof, in an amount and for a time sufficient to treat MS, wherein the VLA-4 blocking antibody is administered at least once when the interferon beta is not present at a therapeutic level in the subject.

## Claims

1. Use of a VLA-4 binding protein for the preparation of a medicament for the treatment of a subject having multiple sclerosis, wherein said treatment comprises:
administering the VLA-4 binding protein to the subject who previously received treatment with a first agent for at least 6 months and who has experienced at least one relapse;
administering the VLA-4 binding protein to the subject when the first agent is not present at a therapeutic level in the subject;
wherein the first agent is selected from the group consisting of an interferon, glatiramer acetate, a fumarate, mitoxantrone, a chemotherapeutic, an immunoglobulin, a statin, azathioprine, and a TNF antagonist.

2. A VLA-4 binding protein for use in the treatment of a subject having multiple sclerosis, wherein said treatment comprises:
administering the VLA-4 binding protein to the subject who previously received treatment with a first agent for at least 6 months and who has experienced at least one relapse;
administering the VLA-4 binding protein to the subject when the first agent is not present at a therapeutic level in the subject;
wherein the first agent is selected from the group consisting of an interferon, glatiramer acetate, a fumarate, mitoxantrone, a chemotherapeutic, an immunoglobulin, a statin, azathioprine, and a TNF antagonist.

3. The use of claim 1 or the VLA-4 binding protein for use according to claim 2, wherein the first agent is an interferon, optionally interferon beta.

4. The use of claim 1 or the VLA-4 binding protein for use according to claim 2, further wherein the VLA-4 binding protein is administered in the absence of a corticosteroid for at least four months.

5. The use of claim 1 or the VLA-4 binding protein for use according to claim 2, wherein the multiple sclerosis is refractory to treatment with the first agent in the subject.

6. The use of any one of claims 1 or 3-5 or the VLA-4 binding protein for use according to any one of claims 2-5, wherein the administration of the VLA-4 binding protein is continued in the absence of the first agent for at least four months, optionally at least eight months.

7. The use of any one of claims 1 or 3-6 or the VLA-4 binding protein for use according to any one of claims 2-6, wherein the subject has not been administered the first agent for at least three months prior to the first administration of the VLA-4 binding protein.

8. The use of any one of claims 1 or 3-6 or the VLA-4 binding protein for use according to any one of claims 2-6, wherein the subject has been administered the first agent within 6 weeks, optionally within 2 weeks, prior to the first administration of the VLA-4 binding protein.

9. The use of any one of claims 1 or 3-6 or the VLA-4 binding protein for use according to any one of claims 2-6, wherein the subject has not been administered a VLA-4 binding protein since being diagnosed with multiple sclerosis.

10. The use of any one of claims 1 or 3-9 or the VLA-4 binding protein for use according to any one of claims 2-9, wherein the VLA-4 binding protein is a VLA-4 binding antibody.

11. The use of claim 10 or the VLA-4 binding protein for use according to claim 10, wherein the VLA-4 binding antibody:
a. inhibits VLA-4 interaction with VCAM-1; and/or
b. binds at least the α chain of VLA-4.

12. The use of claim 10 or the VLA-4 binding protein for use according to claim 10, wherein the VLA-4 binding antibody is natalizumab.

13. The use of claim 10 or the VLA-4 binding protein for use according to claim 10, wherein the VLA-4 binding antibody competes with HP1/2 or natalizumab for binding to VLA-4.

14. The use of any one of claims 10-13 or the VLA-4 binding protein for use according to any one of claims 10-13, wherein the VLA-4 binding antibody is human or humanized.

15. The use of any one of claims 10-14 or the VLA-4 binding protein for use according to any one of claims 10-14, wherein the VLA-4 binding antibody comprises each of the three heavy chain complementarity determining regions (CDRs) of natalizumab and each of the three light chain CDRs of natalizumab.

16. The use of any one of claims 10-14 or the VLA-4 binding protein for use according to any one of claims 10-14, wherein the VLA-4 binding antibody comprises a heavy chain variable domain amino acid sequence that is identical to the heavy chain variable domain amino acid sequence of natalizumab; and/or
the VLA-4 binding antibody comprises a light chain variable domain amino acid sequence that is identical to the light chain variable domain amino acid sequence of natalizumab.

17. The use of any one of claims 1 or 3-16 or the VLA-4 binding protein for use according to any one of claims 2-16, wherein the multiple sclerosis is chronic progressive multiple sclerosis, primary-progressive (PP) multiple sclerosis, secondary progressive multiple sclerosis, or progressive relapsing multiple sclerosis.

18. The use of any one of claims 1 or 3-17 or the VLA-4 binding protein for use according to any one of claims 2-17, wherein the VLA-4 binding protein is administered intravenously to the subject in a plurality of doses, each dose being between 200-400 mg, optionally wherein each dose is 300 mg.

19. The use of claim 18 or the VLA-4 binding protein for use according to claim 17, wherein the plurality of doses is administered every 4 weeks.

20. The use of any one of claims 1 or 3-19 or the VLA-4 binding protein for use according to any one of claims 2-19, wherein the subject is monitored for an improvement in EDSS score.
